# EUROPEAN PATENT APPLICATION

(11) **EP 2 050 458 A1**
(43) Date of publication of application: **22.04.2009**
(21) Application number: 07291268.6
(22) Date of filing: 17.10.2007
(51) Int. Cl.: A61K 38/20, A61P 35/00, A61P 35/02, A61K 31/08, A61K 31/277, A61K 31/427, A61K 31/58, C07K 14/16

(54) **IL24 for inducing hyperproliferative or autoimmune cell death**

(71) Applicant: INSTITUT NATIONAL DE LA SANTE ET DE LA RECHERCHE MEDICALE (INSERM), 75013 Paris (FR)
(72) Inventor: Dalloul, Ali, 54000 Nancy (FR); Gary, Hélène, 92170 Vanves (FR)
(74) Representative: Colombet, Alain André

(57) **Abstract**

The invention concerns the use of IL24 for the treatment of a hyperproliferative or autoimmune disorder by inducing death of hyperproliferative or autoimmune cells, in particular through stimulation of p53 expression. The invention also relates to a a kit comprising IL24 and a cytokine likely to trigger hyperproliferative or autoimmune cells into cell cycle as a combined preparation for sequential use for the treatment of a hyperproliferative or autoimmune disorder. The invention further provides an inhibitor of activated-Stat3 for treating chronic lymphocytic leukaemia.

## Description

The invention concerns the use of IL24 for the treatment of a hyperproliferative or autoimmune disorder by inducing death of cycling hyperproliferative or autoimmune cells, in particular through stimulation of p53 expression. The invention also relates to a kit comprising IL24 and a cytokine which triggers hyperproliferative or autoimmune cells into cell cycle, as a combined preparation for sequential use for the treatment of a hyperproliferative disorder. The invention further provides an inhibitor of activated-Stat3 for treating chronic lymphocytic leukaemia.

Interleukin (IL)-24 belongs to the IL10 family of cytokines, which also includes IL19, IL20, IL22 and IL26 (Kotenko. Cytokine and growth factor reviews. 2002. 13:223-40 ; Renauld. Nat Rev Immunol. 2003. 3:667-76). IL24 was originally described as a gene the expression of which was induced by *in vitro* treatment of melanoma cells with IFN-b + Mezerein (Jiang et al. Oncogene.1995. 11:2477-86; international patent application WO 95/11986). This condition resulted in growth arrest, loss of tumourogenic potential and terminal differentiation of melanocytes, hence the gene name, melanoma differentiation antigen- (MDA)-7.

Subsequent studies demonstrated that an alternative splicing product of MDA-7 encoded a secreted protein and because of sequence homology, chromosome localization and selective expression in lymphoid tissues, it was named IL24. Loss of expression during the process of progression towards malignancy (Ellerhorst et al. J Clin Oncol. 2002.20:1069-74; Ekmekcioglu et al. Int J Cancer. 2001 Oct 1;94(1):54-9; international patent application WO 01/05437) and the finding that IL24-encoding adenovirus vectors induced growth suppression without differentiation in several human cancer cells but not in normal cells (Lebedeva et al. Oncogene. 2002. 21:708-18 ; Su et al. Proc Natl Acad Sci U S A. 1998. 95:14400-5), suggested that it could be an anti-cancer drug.

Among cells of the immune system, IL24 is expressed mostly by T-cells and monocytes at variance with B and NK cells (Poindexter et al. J Leukoc Biol. 2005. 78: 745-52). Forced expression of CD5, a negative regulator of B-cell Receptor signaling (Bikah et al. Science. 1996. 274:1906-9 ; Gary-Gouy et al. J Biol Chem. 2000. 275:548-56 ; Gary-Gouy et al. J Immunol. 2002. 168:232-9), into B-cells was found to augment the expression of IL-10 mRNA and protein (Gary-Gouy et al. Blood. 2002. 100:4537-43). DNA chip analysis revealed that other transcripts of the IL-10 family were also induced by CD5 (Gary-Gouy et al. J Immunol, 2007;179(7):4335-4344).

Importantly, high IL24/MDA-7 mRNA and protein expression was found in 30/30 samples from CD5⁺ chronic lymphocytic leukemia (CLL) B-cells, reminiscent of their mature differentiated/memory status (Sainz-Perez et al. Leukemia. 2006. 20:498-504). The Inventors confirmed that Mda7/IL24 was almost absent in normal adult B-cells (Poindexter et al. J Leukoc Biol. 2005. 78: 745-52). Such high expression in CLL argued against a proapoptotic role for the endogenous IL24 protein, and instead, IL24 mRNA silencing promoted CLL cell apoptosis, in part through inhibition of p38 Mitogen-Activated-Protein kinase (MAPK) phosphorylation (Sainz-Perez et al. Leukemia. 2006. 20:498-504) indicating that IL24 is a survival factor in CLL.

The Inventors have now demonstrated that malignant cells express receptors for IL24 suggesting that they may respond to this cytokine. However, they failed to detect IL24 in sera from 28/28 CLL patients whereas amounts of 100-500 pg/ml are commonly detected in healthy controls. This suggested that leukemic B-cells failed to secrete IL24 thereby diluting normal IL24 producing cells or alternatively, that they prevented normal cells from secreting this cytokine.

The protective effect of endogenous IL24 on CLL cells seemed paradoxical given the apoptotic effect of adenovirus-delivered IL24 on cancer cells (Lebedeva et al. Oncogene. 2002. 21:708-18 ; Su et al. Proc Natl Acad Sci U S A. 1998. 95:14400-5).

The effects of rIL24 on malignant B-cells were therefore studied, and it was found that it exerted an inhibitory effect on thymidine incorporation by IL2-stimulated CLL B-cells. In contrast, rIL24 alone exerted a short term protection on cultured cells. Exploring further its mechanisms of action the Inventors identified that, following stimulation with IL2, rIL24 induced apoptosis of cells engaged into cell cycle.

As CLL is characterized by the *in vivo* accumulation of long-lived and slow dividing monoclonal B-cells arrested at the G0/G1 phase and have a genetic profile related to that of memory B-cells (Klein et al. J Exp Med. 2001. 194:1625-1638; Rosenwald et al. J Exp Med. 2001. 194:1639-47) it is thought that IL24 is protective on resting cells and in terminally-differentiated cells, whereas it is detrimental on proliferating cells.

Without willing to be linked to a mechanism, the pro-apopotic effect of rIL24 likely involves dephosphorylation of phospho-Stat3 and enhancement of p53 expression mediating cell cycle arrest and apoptosis of cells in G2/M cycle phase. This mechanism was unexpected as it is opposite to the one generally attributed to members of the class-II family of cytokines. In addition, at variance with the previously reported IL24-receptor and JAK/STAT-independent action of adenovirus-mda7 (Sauane et al. J Cell Physiol. 2003. 196:334-345), the Inventors demonstrated a specific induction of apoptosis by rIL24.

### Definitions

"IL24" denotes human Melanoma differentiation-associated gene-7/interleukin-24 (MDA-7/IL24) or related genes or proteins identified is other species, such as mouse FISP, or rat C49A/Mob-5.

Human MDA-7/IL24 gene, which was initially identified by Jiang et al. (Oncogene.1995. 11:2477-86), has been deposited in GenBank under accession number U16261 (SEQ ID NO:1). The term "human IL24" refers to a protein having the amino acid sequence set forth as SEQ ID NO:2, having GenPept Accession Number AAA91780.

The term IL24 is used indifferently to designate recombinant, synthetic or native (i.e. isolated endogenous) IL24 gene or protein.

In the context of the invention, "IL24" further includes fragments or variants of IL24, in particular of human IL24.

A nucleic acid sequence "encoding" human IL24 may have the coding sequence as set forth in SEQ ID NO:1 or another sequence which, when translated, produces a protein having the same amino acid sequence as set forth in SEQ ID NO:2. This includes "sequence-conservative variants" of SEQ ID NO:1, or of IL24 CDS (nucleotides 275 to 895 of SEQ ID NO:1), i.e. polynucleotides in which a change of one or more nucleotides in a given codon position results in no alteration in the amino acid encoded at that position.

As used herein an "IL24 variant" refers to a polypeptide having at least about 80 percent, or at least about 85, 90, 95, 97 or 99 percent sequence identity with the sequence of human IL24 (SEQ ID NO:2). Percent identity may be calculated by sequence alignment over the defined length of the sequences (in particular full length of the shorter sequence) using, for example, the GCG (Genetics Computer Group, Program Manual for the GCG Package, Version 7, Madison, Wisconsin) pileup program, or by sequence comparison algorithms, such as NCBI BLAST or FASTA.

IL24 variants may be "function-conservative variants", i.e. variants in which a given amino acid residue has been changed without altering the overall conformation and function of the IL24 polypeptide, including, but not limited to, replacement of an amino acid with one having similar properties (such as, for example, polarity, hydrogen bonding potential, acidic, basic, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Such changes are expected to have little or no effect on the apparent molecular weight or isoelectric point of the protein or polypeptide. Amino acids other than those indicated as conserved may differ in a protein or enzyme so that the percent protein or amino acid sequence similarity between any two proteins of similar function may vary.

Fragments of IL24 with anti-proliferative activity have been described in the art. For instance, US patent application 2006/0292157 described antiproliferative IL24 fragments consisting of amino acid residues 48-104, 48-206, 63-101 104-206, 105-154, or 159-201 of SEQ ID NO:2.

"Stat3" denotes Signal Transducer and Activator of Transcription 3. STATs, which are present in the cytoplasm of cells under basal conditions, are activated by phosphorylation on a single tyrosine residue located towards the carboxy terminus of the protein (phosphorylation on Tyr705 in the case of Stat3). Phosphorylation of Stat3 elicits dimerization of two Stat3 molecules via reciprocal pTyr705-SH2 domain interactions then translocation to the nucleus, thereby regulating transcription. Stat3 targets genes which are involved in fundamental events of tumor development, e.g. proliferation, survival, self-renewal, invasion and angiogenesis. Stat3 was reported to repress p53 gene expression (Niu et al. Mol Cell Bio. 2005. 25: 7432-40). In some cases, phosphorylation of Stat3 on a further residue (Ser727) has been reported to enable maximal transcriptional activity of Stat3.

Thus, as used herein, a "Stat3-activated cell" is meant for a cell which contains Stat3 phosphorylated on Tyr705 (also referred to as "activated-Stat3", "phosphorylated-Stat3", "phospho-Stat3", "P-Stat3" or "pY705-Stat3"). In cells, Stat3 phosphorylation may be induced by cell exposure to cytokines or growth factors. Phosphorylation may be mediated by Jak family kinases associated with cytokines receptors (such as IFN alpha/beta, IL-6, IL-12,), growth factor receptors with intrinsic tyrosine kinase activity (such as EGF, PDGF), or non-receptor kinases such as Src family kinases. Under normal physiologic conditions, Stat3 activation in response to growth factors or cytokines is a transient response. However, constitutive or continuous Stat3 activation is observed in tumour cells, e.g. through enhanced tyrosine kinase activity that mediates Stat3 phosphorylation and/or by loss of repression of the mechanisms that normally silence Stat3 response.

An "inhibitor of activated-Stat3" denotes an agent capable of inhibiting activated-Stat3 function, of blocking Stat3 phosphorylation, or of inducing dephosphorylation of phosphorylated-tyrosine705 of Stat3 (pY705-Stat3).

The inhibitor may be a direct or indirect Stat3 inhibitor. A direct Stat3 inhibitor inhibits the protein by direct binding, whereas an indirect Stat3 inhibitor refers to agents that result in reduced activation of Stat3 by indirect means (i.e. Stat3 pathway inhibitor).

In the context of the invention, the term "treating" or "treatment", as used herein, means reversing, alleviating, inhibiting the progress of, or preventing the disorder or condition to which such term applies, or one or more symptoms of such disorder or condition.

"Therapeutically effective amount" means an amount of a therapeutic agent or pharmaceutical composition according to the present invention effective in producing the desired therapeutic effect.

According to the invention, the term "patient", or "patient in need thereof', is intended for a human or non-human mammal affected or likely to be affected with a hyperproliferative cell disorder. Preferably, the patient is a human. Preferred non-human mammals include rodents, such as mouse or rat, feline and canine.

### Treatment of hyperproliferative disorders with IL24

The Inventors have demonstrated that whereas rIL24 enhanced cell survival in chronic lymphocytic leukemia (CLL) B-cells, sequential incubation with rIL2 and rIL24 induced apoptosis of CLL cells into S and G2/M cell cycle phase. The mechanism involved an increase in p53 transcription and protein in parallel to recruitment of (SHP)-1 phosphatase to the IL24 receptor and dephosphorylation of phospho-Stat3 which is known to be a p53 transcriptional repressor. The effect of IL24 on P-Stat3 was reversed by pervanadate and mimicked by troglitazone, a PTP1B phosphatase activator, which inhibited P-Stat3 and augmented p53. IL24-induced apoptosis was reversed by pharmacological inhibitors of phosphatase activity, by the p53 inhibitor pifithrin-alpha and partly by the caspase inhibitor ZVAD. Sequential activation with IL2 followed by inhibition of P-Stat3 and induction or stabilization of p53 is therefore useful in therapy of chronic lymphocytic leukemia.

More generally, the results disclosed herein provide for a treatment of hyperproliferative disorders with IL24 by inducing death of proliferating or cycling hyperproliferative cells

These results also open the way to the use of IL24 for inducing death of proliferating or cycling autoimmune lymphocytes, in particular B cells, associated with autoimmune disorders.

Accordingly, the invention relates to the use of IL24 for the treatment of a hyperproliferative or autoimmune disorder, by inducing death of cycling hyperproliferative or autoimmune cells.

The invention also provides a method of treating a hyperproliferative or autoimmune disorder in a patient in need thereof, wherein said patient is administered with IL24 to induce death of cycling hyperproliferative or autoimmune cells associated with the hyperproliferative or autoimmune disorder.

More particularly, administration of IL24 induces death of the hyperproliferative or autoimmune cells where said cells were beforehand triggered into cell cycle by administering the patient with a cytokine, especially a cytokine likely to induce cycling and/proliferation of said hyperproliferative or autoimmune cells.

The time frame for sequential administration of the cytokine, then of IL24, may be readily determined by the skilled person. For instance, a cytokine could be administered at least two days, at least one day, or 24, 16, 12, 8 or 4 hours prior to administering IL24. Typically, the cytokine could be administered to the patient 24 hours before administering said patient with IL24.

As used herein, the term "cytokine" is used indifferently to denote interleukins, chemokines and growth factors.

A "cycling cell" may be generally defined as a cell which may be found into the S and/or G2/M phase within a 24-hour period of time.

A cytokine likely to induce cycling and/or proliferation of a given hyperproliferative or autoimmune cell type may be readily chosen by the skilled in the art. Suitable cytokines will be in particular those which are considered as natural growth-factors for a specific hyperproliferative or autoimmune cell, e.g. cytokines which expression is found to be induced or enhanced in said hyperproliferative or autoimmune cell by comparison with a normal cell of the same type.

Examples of cytokines likely to induce proliferation of some hyperproliferative cells are described hereafter.

| **Cytokine** | **Hyperproliferative disorder** |
|---|---|
| IL-6 | Myeloma, hepatocarcinoma, ER-α-positive breast cancer* <*Sasser et al, FASEB J. 2007 Jun 22> |
| IL2 | Chronic lymphocytic leukaemia |
| EGF | Breast, oesophageal, bladder, prostate, biliary tract, head & neck, liver, lung, prostate, colorectal, astrocytoma, glioblastoma |
| VEGF | Melanoma, mesothelioma, prostate, angioblastoma, bladder, thyroid, breast, colorectal, kidney, oesophageal, astrocytoma, glioma |
| PDGF | Prostate, gastro-intestinal (GIST), breast |
| IGF | Melanoma |
| HGF | Melanoma, breast |
| TGF-beta | breast, oesophageal |

It is thought that once the hyperproliferative or autoimmune cells have been prompted to cycle and proliferate, then exposure of the cells to IL24 induces death by stimulating p53 expression.

"Stimulating p53 expression" may be achieved, for instance, by enhancing p53 basal expression, in particular by derepressing (i.e. suppressing repression of) p53 expression, by stabilizing p53 mRNa and/or protein, by inhibiting degradation of p53 mRNa and/or protein, or by any combination of these effects. As used herein "p53 expression" denotes expression of p53 mRNa and/or protein. Preferably, stimulation of p53 protein expression is achieved. The skilled in the art may readily determine by conventional means if IL24 elicits stimulation of p53 mRNa and/or protein expression, for instance by measuring and comparing levels of p53 mRNa and/or protein detected in hyperproliferative cells of a patient before and after exposure to IL24.

A "hyperproliferative disorder" generally denotes a disease associated with cells which have hyperproliferative capacity, which is either constitutive (activated cells which are continuously cycling) or inducible (resting cells triggered into hyperproliferation by exposure to a cytokine or growth factor).

In the context of the invention, hyperproliferative and autoimmune cells are preferably cells in which the p53 protein, when expressed, is functional, i.e. cells that contains wild-type p53 gene or a p53 gene with silent mutations.

IL24 may be used in particular for the treatment of a hyperproliferative or autoimmune disorder wherein hyperproliferative cells are Stat3-activated cells, especially continuously Stat3-activated cells. Stat3-activated cells may be readily identified by the skilled person through detecting Stat3 phosphorylation in the cells. For instance phosphorylation of STAT3 on tyrosine705 can be detected by immunocytochemistry, immunohistochemistry and/or flow cytometry using antibodies which specifically recognize this modification. Monoclonal or polyclonal antibodies directed against pY705-Stat3 are commercially available. Furthermore, development of antibodies specifically directed against pY705-Stat3 is within the ordinary skills of the skilled in the art. Reference can be made for instance to Köhler and Milstein (Nature. 1975 Aug 7;256(5517):495-7).

According to an embodiment, death of hyperproliferative or autoimmune cells is induced by derepression, or loss of repression, of p53 expression. In particular, this effect may be mediated by dephosphorylation of phospho-Stat3 (p-Stat3), i.e. dephosphorylation of Tyr705 of Stat3 (pY705-Stat3).

Stat3-activation of the hyperproliferative or autoimmune cells may be constitutive. Indeed Stat3 has been reported to be activated inappropriately in numerous cancers, for instance without any limitation, in haematologic malignancies such as acute myelogenous leukaemia (AML), multiple myeloma, Hodgkin's disease, non-Hodkin's lymphoma, B cell, cutaneous T cell lymphoma, or in non-hematologic malignancies such as breast, prostate, lung (non-small cell), head and neck (squamous cell), ovarian, and pancreatic cancer, melanoma, hepatocellular carcinoma and cholangiocarcinoma (Frank, Cancer Letters 251 (2007) 199-210).

Alternatively, Stat3-activation may have been induced in the hyperproliferative or autoimmune cells by exposure to a cytokine. In particular, cytokines likely to induce cycling and/or proliferation of hyperproliferative or autoimmune cells may act by inducing Stat3 phosphorylation. Examples of cytokines or growth factors capable of inducing Stat3-activation include IFN alpha/beta, IL-6, IL-12, IL2, EGF, PDGF.

Cell exposure to a cytokine or growth factor may be performed for instance by administering the patient to be treated with an appropriate cytokine, whereby hyperproliferative cells are contacted with the cytokine.

For instance, where the hyperproliferative cells are chronic lymphocytic leukaemia cells, cells may be contacted beforehand with IL2, whereby Stat3 phosphorylation is induced. Where the hyperproliferative cells are myeloma cells, the cells may be contacted beforehand with IL-6. Where the hyperproliferative cells are epidermoid cancer cells, such as lung, breast or ovary cancer cells, the cells may be contacted beforehand with EGF.

In the chronic lymphocytic leukaemia (CLL) model used by the Inventors to decipher the role of IL24, it was observed that the caspase inhibitor ZVAD did not reverse completely death induced by IL24 in IL2-activated CLL cells, suggesting that cell death is not mediated by apoptosis only. Accordingly, in the context of the invention, hyperproliferative au autoimmune cell death, in particular cell death triggered by p53 stimulation, may be achieved by apoptosis and/or senescence.

According to an embodiment, the hyperproliferative disorder may be cancer, such as a haematologic cancer, in particular acute myelogenous leukaemia (AML), chronic lymphocytic leukaemia (CLL), multiple myeloma, Hodgkin's disease, non-Hodkin's lymphoma, B cell, cutaneous T cell lymphoma, or a non-haematologic cancer, for instance brain, epidermoid (in particular lung, breast, ovarian), head and neck (squamous cell), bladder, gastric, pancreatic, head, neck, renal, prostate, colorectal, oesophageal or thyroid cancer, and melanoma.

Preferably a cancer according to the invention may be selected from the group consisting of chronic lymphocytic leukaemia (CLL), myeloma, epidermoid cancer.

According to another embodiment, the hyperproliferative disorder may be a non-cancerous hyperproliferative disorder such as benign hyperplasia of the skin (e.g., psoriasis) or prostate (e.g., benign prostatic hypertrophy (BPH)), rheumatoid arthritis, inflammatory bowel disease, osteoarthritis, leiomyomas, adenomas, lipomas, hemangiomas, fibromas, vascular occlusion, restenosis, atherosclerosis, or oral hairy leukoplakia.

The autoimmune disorder may be an autoimmune B or T cell disorder, preferably B cell, such as systemic erythematosus Lupus, Sjogren's syndrome and rheumatoid arthritis.

Preferably, the hyperproliferative or autoimmune disorder according to the invention is associated with hyperproliferative or autoimmune cells, in particular cancer cells, that express an IL24 receptor, in particular a functional IL24 receptor. Two heterodimeric receptor complexes have been reported for IL24: IL20R1/IL20R2 and IL22R/IL20R2.

In order to implement the treatment according to the invention, IL24 protein or a nucleic acid, such as DNA or RNA, encoding IL24 protein under the control of a promoter operable in eukaryotic cells, may be administered to the patient in need thereof. The nucleic acid encoding IL24 may consist in a vector, i.e. a vehicle by which a DNA or RNA sequence can be introduced into a host cell, in particular a hyperproliferative cell, so as to transform the host cell and promote expression of the sequence encoding IL24.

Methods to introduce a nucleic acid molecule into cells are well known in the art. For instance a naked nucleic acid molecule may be introduced into the cell by direct transformation. Alternatively, the nucleic acid molecule may be embedded in liposomes. Accordingly, a nucleic acid encoding IL24 may be introduced into the cells by naked DNA technology, adenovirus vector, adeno-associated virus vector, Epstein-Barr virus vector, Herpes virus vector, attenuated HIV vector, retroviral vectors, vaccinia virus vector, liposomes, antibody-coated liposomes, mechanical or electrical means, or any other suitable method known by the skilled in the art.

Preferably, IL24 protein or nucleic acid may be administered in the form of a pharmaceutical composition.

A pharmaceutical composition comprises IL24 protein or a nucleic acid sequence encoding IL24 together with a pharmaceutically acceptable carrier.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable carrier" includes any diluents, adjuvants, excipients, or vehicles, such as preserving agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. Methods of formulation are well known in the art and are disclosed, for example, in Remington: The Science and Practice of Pharmacy, Gennaro, ed., Mack Publishing Co., Easton, Pa., 19th ed., 1995.

For pharmaceutical use, IL24 protein may be formulated for any suitable route of administration, for instance for topical or parenteral, particularly intravenous or subcutaneous, delivery according to conventional methods. IL24 may preferably be used in a concentration of about 10 to 100 µg/ml of total volume, although concentrations in the range of 1 ng/ml to 1000 µg/ml may be used. For topical application, the protein may be applied in the range of 0.1-10 _{P}g/_{CM2}, with the exact dose determined by the clinician according to accepted standards, taking into account the nature and severity of the condition to be treated, patient traits, etc. Determination of dose is within the level of ordinary skill in the art. Dosing is daily or intermittently over the period of treatment. Intravenous administration may be performed be by bolus injection or infusion over a typical period of one to several hours. Sustained release formulations can also be employed. In general, a therapeutically effective amount of IL24 is an amount sufficient to produce a clinically significant change in the treated condition, such as a clinically significant change in cell hyperproliferation, or a significant reduction in morbidity.

### Kits

The invention also relates to a kit comprising IL24 and a cytokine likely to trigger a hyperproliferative or autoimmune cell type into cell cycle, as a combined preparation for sequential use for the treatment of a disorder associated with said hyperproliferative or autoimmune cell.

Said cytokine may induce Stat3 phosphorylation.

In particular, said cytokine may be IL2 and said kit is intended for sequential use for the treatment of chronic lymphocytic leukaemia.

Said cytokine may also be IL-6 and said kit is intended for sequential use for the treatment of myeloma.

Said cytokine may also be EGF and said kit is intended for sequential use for the treatment of an epidermoid cancer cells, such as lung, breast and ovary cancer.

The suitable regimen for sequential administration of the cytokine or growth factor, then of IL24, may be readily determined by the skilled person. For instance, a cytokine or growth factor could be administered at least two days, at least one day, or 16, 12, 8 or 4 hours prior to administering IL24.

### Treatment of chronic lymphocytic leukaemia with inhibitors of activated-Stat3

Additionally, demonstration that IL24 can induce death of chronic lymphocytic leukaemia (CLL) cells via the Stat3/p53 pathway more generally identifies Stat3 as a target in the treatment of CLL.

Accordingly, the invention also relates to the use of an inhibitor of activated-Stat3 for the manufacture of a medicament for treating chronic lymphocytic leukaemia.

The invention further provides an inhibitor of activated-Stat3 for treating chronic lymphocytic leukaemia.

A method of treating chronic lymphocytic leukaemia which comprises administering a patient in need thereof with an inhibitor of activated-Stat3 is also contemplated.

Unless otherwise specified, the definitions given above also apply to this section.

As CLL is characterized by the *in vivo* accumulation of long-lived and slow dividing monoclonal B-cells arrested in the G0/G1 phase, preferably, prior to the treatment with said a medicament or inhibitor of activated-Stat3, CLL cells are contacted with a cytokine or growth factor, in particular with IL2, to induce cell proliferation and Stat3-phosphorylation. Accordingly, afterwards administration of the medicament or inhibitor of activated-Stat3 can induce death of cycling CLL cells, i.e. cells which have been triggered to enter in S and G2/M cell cycle phases by IL2.

The inhibitor may be a direct or indirect Stat3 inhibitor.

The inhibitor or agent may be of any nature, for instance a small molecule, an organic or inorganic compound, a polypeptide or protein (e.g. peptide inhibitors, peptdidomimetics, antibodies), or a nucleic acid (e.g. antisense, RNA interference, aptamers).

An agent capable of directly inhibiting activated-Stat3 function may act in particular by preventing dimerization of phosphorylated-Stat3 (P-Stat3) and/or translocation of P-Stat3 dimer into the nucleus and/or binding to a gene which transcription is regulated by Stat3.

For instance, specific protein inhibitors of activated STAT (PIAS), and in particular of Stat3 (PIAS3), that block the DNA binding activity of a specific STAT have been described in the art. For a description of PIAS3, reference may be made in particular to patent US 7,265,202, and article Chung et al. (Science. 1997;278(5344):1803-5).

Furthermore, 6-nitrobenzo[b]thiophene-1,1-dioxide, also called Stattic (Stat3 three inhibitory compound), is an inhibitor of Stat3 selective over Stat1 and Stat5, which inhibits phosphopeptide binding to the SH2 domain of Stat3 (McMurray. Chemistry & Biology. 2006. 13(11): 1123-1124).

Stat3 pathway inhibitors have been described in the art. Reference may be made for instance to cucurbitacin I (Blaskovich et al. Cancer Res. 2003. 63(6):1270-9), cucurbitacin Q (Sun et al. Oncogene. 2005. 24(20):3236-45) afterwards identified as a mixture withacnistin, 3-methoxy-2,3-dihydrowithacnistin, and 3-ethoxy-2,3-dihydrowithacnistin (published US patent application US 2007/191490), and Withacnistin (published US patent application US 2007/191490).

Besides, curcumin has been demonstrated to be an inhibitor of STAT3 phosphorylation (Bharti et al. J Immunol. 2003;171(7):3863-71).

AG490 (α-Cyano-(3,4-dihydroxy)-N-benzylcinnamide) has also been reported to be an inhibitor of STAT3 phosphorylation, probably through inhibition of the JAK2 kinase that phosphorylates STAT3 (Meydan et al. Nature. 1996. 379:645).

Additionally, troglitazone is an activator of protein-tyrosine phosphatase (PTP)-1 B, which subsequently reduces phosphotyrosine 705 STAT3 (pY705-STAT3) (Akasaki et al. J Biol Chem. 2005. 281:6165-74). Troglitazone was herein found to induce dephosphorylation of P-Stat3 and cell death in IL2-activated CLL cells.

Accordingly, an inhibitor of activated-Stat3 may be selected from the group consisting of IL24, troglitazone, PIAS3, 6-nitrobenzo[b]thiophene-1,1-dioxide, cucurbitacin I, cucurbitacin Q, Withacnistin, curcumin, and AG490.

Inhibitors of activated-Stat3 may be administered in the form of a pharmaceutical composition. Such pharmaceutical compositions comprise an inhibitor of activated-Stat3 together with a pharmaceutically acceptable carrier, as defined above.

The invention will be further illustrated in view of the following figures and examples.

### FIGURES

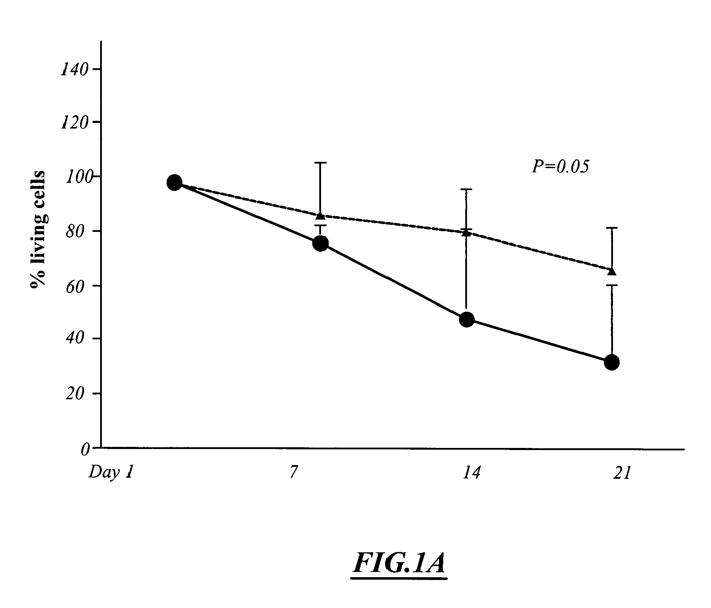

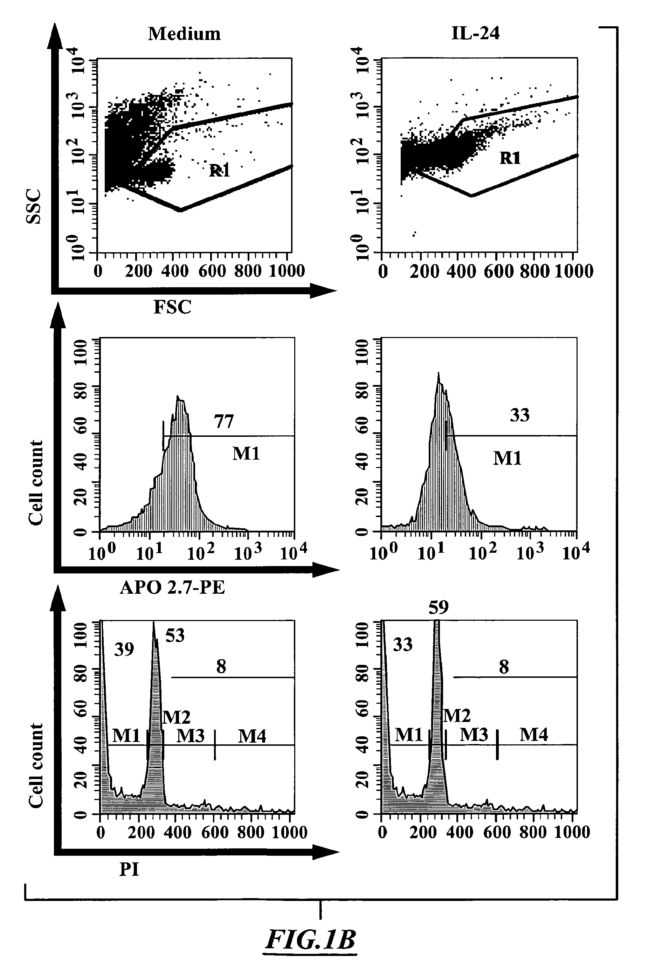

Figure 1. rIL24 protects CLL B-cells from death in culture.
   a) CLL cells (2x10⁶/ml) were cultured in complete medium (•) or with 100 ng/ml rIL24 (▲) every 7 days and cells counted using trypan blue exclusion. % living cells was calculated in each sample (mean ± SD, n=8). b) Cell scatter, Apoptosis shown by Apo 2.7-PE staining, and cell cycle shown by PI staining from one representative sample (CLL 70) at day 21. M1: hypodiploid (apoptotic cells) M2: cells into G0/G1, M3: cells into S+G2+M, M4: hyperdiploid cells.
Figure 2. rIL24 inhibits thymidine incorporation in activated CLL cells.
   a) IL24 was added to IL2-cultured cells as in figure 1, one day before (d-1) at the same time (d0) or one day after (d=1) IL2. b) IL24 was added to CLL cells cultured for 24 h with IL2, IL2+CD40-Ligand or anti-IgM+CD40-Ligand. Tymidine (0.5 mCi/ml) was added at day 3 for 15 h. (n=5 to 8)
Figure 3. rIL24 induces apoptosis and decreases the population of CLL cells engaged into S/G2/M cell cycle phase.
   a) A representative sample (CLL) was synchronized with cold thymidine, and cultured in Medium only or supplemented with IL24 or IL2 for 24 h, then IL24 for 2 days and cells fixed and stained with PI. M1: hypodiploid cells (dead), M2: cells in G0/G1, M3: cells in S+G2/M, M4: hyperdiploid cells. The increase in M3+M4 population under IL2, and the decrease of this population under IL2+IL24 together with the increase in the M1 population, are shown. b) Cell cycle analysis. Mean ± SD (n=8) of the M1 to M4 populations calculated as in (a) from CLL cells cultured with IL2 (hatched histogram) or IL2 then IL24 (white histogram). Both conditions were compared using Student's t test. c) Upper: CLL cells were cultured as above in CFSE and IL2 or IL2+IL24 and stained with Apo 2.7 Ab. Lower: Cells cultured for 3 d with IL2 or IL2+IL24 were stained with Annexin-V.

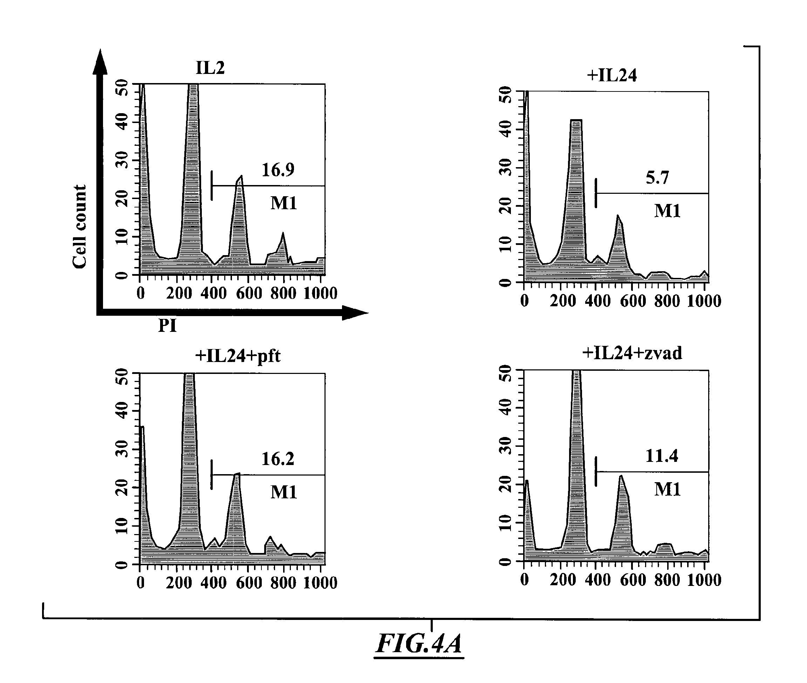

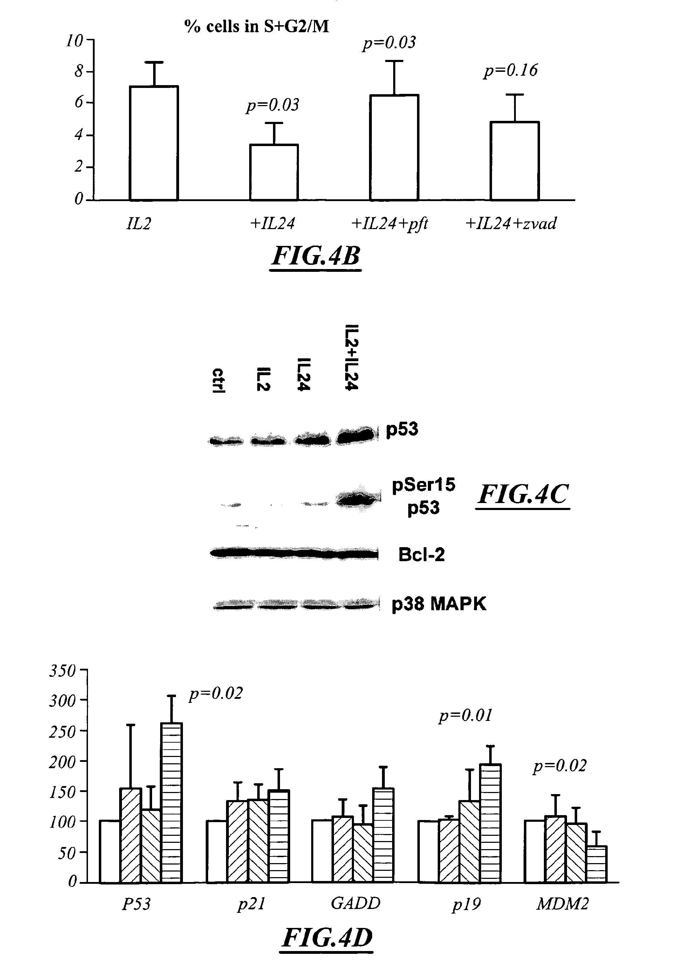

Figure 4. The decrease in S/G2/M population in IL2+IL24-cultured cells is dependent on IL24-mediated induction of p53 expression.
   a and b: the pharmacological inhibitor of p53, pifithrin-alpha (pft) reverses the IL24-mediated decrease of cells engaged into cell cycle a) Cells were cultured overnight with IL2 then incubated with IL24 or with IL24+pft or IL24+zvad-fmk (zvad) for 24 h and stained with PI for cell cycle analysis. % of cells in S+G2+M cello cycle phase is shown, representative experiment from CLL no 44.
   b) % of cells in S+G2+M cell cycle phase of CLL cells cultured as in (a). Mean ± SD from 8 different patients.
   c) Western blot of cells cultured for 3 d shows an increase in total p53 and in p53 phosphorylated on serine-15 residue.
   d) Relative gene expression in CLL cells cultured for 48 h respectively with Medium, IL24, IL2, and IL2+IL24. RNA was extracted at day 3, reverse-transcribed, amplified and analyzed by real-time PCR. Mean ± SD samples from 6 different patients. Values of medium vs IL2+IL24-treated samples were compared using student's t test and shown only when significant (p<0.05).

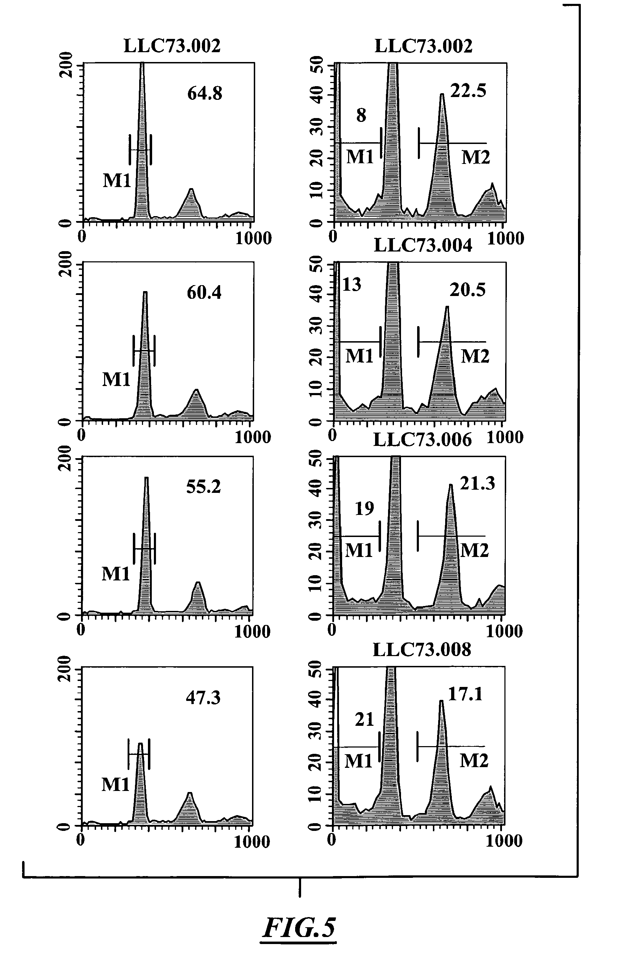

Figure 5. Opposite effects of Troglitazone (TG) on cell death in CLL. Cell cycle analysis performed on CLL cells cultured with IL2 and 0, 5, 20 or 40 µM Troglitazone. TG inhibits predominantly the population in G1 cell cycle phase. Left: analysis of the population in G1, right: analysis of the hypodiploid cells and the cells in G2+M phase.

### EXAMPLES

### Example 1: Material and Methods

### 1.1 Patients

The patients enrolled in the study were diagnosed according to cytologic and immunologic analyses and followed-up at the Percy Military hospital (Clamart, France) and Antoine Beclère Hospital (Clamart, France) between 2004 and 2006. Treatment (if any) free period was at least three months. Approval for these studies was obtained from the institutional review board of the University Hospital Antoine Béclère (Paris XI). All patients gave informed consent. B lymphocytes from leukemic donors were purified and maintained in culture as previously described (Sainz-Perez et al. Leukemia. 2006. 20:498-504). For B cell purification a negative B cell selection was applied using "B-cell enrichment" RosetteSep kit (Stem Cell). The mean purity of B-cells was 96% in donors and 92.5% (89.5% to 98%) in patients.

### 1.2 Reagents

p53 transactivation activity inhibitor (Pifithrin a), caspase inhibitor (ZVAD-fmk, and troglitazone), Protein tyrosine phosphatase inhibitor I (a-Bromo-4-hydroxyacetophenone 4-hydroxyphenacetyl Br), and Protein tyrosine phosphatase inhibitor III (a-Bromo-4-(carboxymethoxy)acetophenone 4-(carboxymethoxy) phenacetyl Br), were all from Calbiochem (Fontenay sous Bois, France). Na-Pervanadate was prepared by mixing equal concentrations of Na₃VO₄ with H₂O₂ (100 mM), both from Sigma, for 15 min at RT, and serial dilutions added to cells.

### 1.3 Cell culture, proliferation, cell cycle and apoptosis

PBMC from patients were cultured in RPMI medium supplemented with 10% FCS or 10% autologous serum, Penicilline (100 U/ml), Streptomycine (100 mg/ml), 2 mM L-glutamate and 1 mM sodium pyruvate (Invitrogen, Cergy, France). Cells were incubated with r-IL2, r-IL24, (all from R&D systems, Lille, France) at respective final concentrations of 50 ng/ml and 100 ng/ml.

Proliferation assays were performed in flat-bottom 96-well plates. Cells (10⁵/200 µl x well) were incubated at 37°C under various conditions. Each condition was done in triplicate. After 72 hours 0,5 µCi of Thymidine Methyl 3H was added to each well and incubated 18 hours at 37°C. Plates were then harvested in a WALLAC Printed Filternat A with a Harvester 96 TOMTEC and let it dry for at least 1 hour. Dried Printed Filternat were put into WALLAC Sample Bag and 10 ml of Betaplate Scint added. Incorporation of thymidine was measeared with the WALLAC 1205 Betaplate Liquid Scintillation Counter (all components are from Perkin Elmer, Courtaboeuf, France).

For apoptosis studies, cells were stained either with the Apo 2.7-PE mAb (Beckman-Coulter, Villepinte, France) on ice for 15 min, washed and resuspended on 300 µl of PBS 1X supplemented with 0,1% BSA or with the Annexin V-FITC apoptosis detection kit (Becton Dickinson Biosciences, Le-Pont-de-Claix, France), 1x10⁵ cells in 100 µl 1X binding buffer were stained with 5 µl of Annexin V-FITC and 5 µl Propidium Iodide (PI) in the dark for 15 min at room temperature (RT). 400 µl of 1X binding buffer was added before fluorescence-activated cell sorted (FACS) analysis. For cell cycle analysis 1x10⁶ cells were washed with cold PBS1X and resuspended in 1ml EtOH 70% and kept on ice for at least 10 min. Cells were then centrifuged and the pellet resuspended in 1ml PBS 1X + RNAse (40 µg) Saint-Quentin Fallavier, France) + Propidium Iodide (18 µl) at room temperature. For carboxy-fluorescein diacetate succinimidyl ester (CFSE) staining 5x10⁶ cells were washed twice in cold PBS and resuspended in PBX + CFSE (5 µM) for 10 min at RT and reaction blocked with addition of FSC (2%), washed twice and resuspended in culture medium at different conditions.

Fluorescence was measured using a FACScan flow cytometer (BD Biosciences), and data analysis was performed using CellQuest software (BD Biosciences).

### 1.4 Immunoprecipitation and Western blot

CLL cells were processed from fresh blood and washed at room temperature in a serum-free RPMI-10 mM Hepes. CLL cells were stimulated or not with rIL24, rIL2 or rIL2 + rIL24 for 48h and lysed. Cells (10x10⁶ CLL) were lysed at 4°C for 30 min in lysis buffer (20 mM tris-Hcl, pH 7.5, 140 mM NaCl, 1 mM EDTA, 50 U/ml aprotinin, 1 mM PMSF, 1 mM sodium orthovanadate) containing 1% nonidet-P-40 detergent. Proteins were separated on SDS-PAGE gels, electrotransferred onto PVDF membranes (Amersham), and blotted with anti-p53 MAb (Dako) or anti-phospho-p53 (S15) MAb (R&D Systems) or anti-p38 (A-12) MAb (Tebu-bio) or anti-Bcl2 (C-2) MAb (Tebu-bio). Blots were revealed using Goat-anti-Mouse HRP-conjugated (Bio-Rad Laboratories) and ECL detection (Amersham).

Lysates from fresh blood CLL cells, stimulated with IL2 at different times, were precipitated with 1 micro g anti-phosphotyrosine clone 4G10 MAb (Zhan et al. Cancer Res. 1994. 54:2755-60) (Upstate, Lake Placid, NY) + protein G sepharose beads (Sigma). Proteins were separated on SDS-PAGE gels, electrotransferred onto PVDF membranes (Amersham), and blotted with anti-STAT3, STAT5, Jak1, or Tyk2 MAbs (BD Transduction Laboratories) . Blots were revealed using Goat-anti-Mouse HRP-conjugated (Bio-Rad Laboratories) and ECL detection (Amersham).

For pervanadate experiments, fresh CLL cells were preincubated or not with IL2 overnight, then incubated or not with pervanadate 25 µM during 5 min and stimulated or not with IL24 for 10 min and lysed as previously described. Blots were hybridyzed with anti phospho-Tyr 705-STAT3 (B-7) MAb (Tebu-bio), dehybridyzed and reblotted with anti STAT3 MAb (BD Transduction Laboratories).

For SHP-1 experiments, lysates in lysis buffer containing 1% Brij 96 detergent were obtained from fresh CLL cells preincubated with IL2 overnight then stimulated with IL24 during 10 min, and precipitated with 1 µg anti-human (IL20 Ralpha + IL20 Rbeta) goat polyclonal antibodies (R&D System) + protein A sepharose beads (Sigma). Blots were hybridyzed with anti-SH-PTP1 (C-19) rabbit polyclonal (Tebu-bio), or anti-human IL20 Ralpha goat polyclonal antibodies (R&D System), and revealed with Goat-anti-rabbit HRP-conjugated or Donkey anti-goat HRP-conjugated (Bio-Rad Laboratories) respectively and ECL detection (Amersham).

### 1.5 Real time PCR

Cells (2 x 10⁶/ml) were incubated in complete medium for 24 h under various conditions, and RNA purified using the RNeasy Mini Kit (Qiagen). Reverse transcription was done with random hexamers using Superscript first strand synthesis system for RT-PCR (Invitrogen). Transcript expression was analyzed by PCR using the TaqMan technology as described (el-Deiry et al. Cell. 1993. 19:817-25). Results were analyzed using the ABI Prism 7700 sequence detection system software (PE Applied Bio System). Each reaction was normalized by the Cycle threshold (Ct) of GAPDH cDNA expression. Relative expression was calculated by measuring the difference of normalized Ct between condition A versus condition B and applying the following formula: difference of expression between A and B = 2e-(CtA-CtB). The TaqMan specific primers and probes described in Table 1 were selected using Primer Express Software and specificity verified using NCBI Blast software.

**Table 1: Primers and probes for real-time PCR**

| **Name** | **Amplicon size** | **Primer Sequence** |
|---|---|---|
| p53 | 82 | L1: ATCTACTGGGACGGAACAGC (SEQ ID NO:3) |
| | | R1: GCGGAGATTCTCTTCCTCTG (SEQ ID NO:4) |
| | | P1: CGGTCTCTCCCAGGACAGGCA (SEQ ID NO:5) |
| p21 | 73 | L1: CGACTGTGATGCGCTAATG (SEQ ID NO:6) |
| | | R1: TCTCGGTGACAAAGTCGAAG (SEQ ID NO:7) |
| | | P1: CATCCAGGAGGCCCGTGAGC (SEQ ID NO:8) |
| p19 | 70 | L1: TCCATCTGGCAGTTCAAGAG (SEQ ID NO:9) |
| | | R1: GCGATGGAGATCAGATTCAG (SEQ ID NO:10) |
| | | P1: TGCCAGAAAGCTGACCACAGCA (SEQ ID NO:11) |
| GADD45a | 114 | L1: GATAACGTGGTGTTGTGCCT (SEQ ID NO:12) |
| | | R1: GGATGTTGATGTCGTTCTCG (SEQ ID NO:13) |
| | | P1: CTGTCGTCGTCCTCGTCCGC (SEQ ID NO:14) |
| MDM2 | 143 | L1: TGCCAAGCTTCTCTGTGAAA (SEQ ID NO:15) |
| | | R1: TTTGATCACTCCCACCTTCA (SEQ ID NO:16) |
| | | P1: CCTGAGTCCGATGATTCCTGCTGA (SEQ ID NO:17) |
| UBC | 98 | L1: CGAGAATGTCAAGGCAAAGA (SEQ ID NO:18) |
| | | R1: AGGGTACGACCATCTTCCAG (SEQ ID NO:19) |
| | | P1: CCGGCAAAGATCAACCTCTGCTG (SEQ ID NO:20) |
| GAPDH | 225 | L1: GAAGGTGAAGGTCGGAGTC (SEQ ID NO:21) |
| | | R1: GAAGATGGTGATGGGATTTC (SEQ ID NO:22) |
| | | P1: CAAGCTTCCCGTTCTCAGCC (SEQ ID NO:23) |

### Example 2: Results.

### 2.1 r IL24 enhances CLL cell survival in culture.

Inhibition of endogenous Mda7/IL24 by siRNA was previously found to be detrimental to the survival of CLL cells (Sainz-Perez et al. Leukemia. 2006. 20:498-504). Therefore, it was investigated whether rIL24 protected CLL cells from death in culture.

Cells from 8 patients were cultured with or without 50 to 300 ng/ml rIL24 every 7 days. In all samples, cell survival after 28 days was significantly augmented by IL24, as evidenced by cell count, (p=0.05) (Fig 1a) and cell cycle analysis. As shown from one representative sample, a significant cell fraction was alive after 21 days of culture with 100 ng/ml of rIL24, whereas most cells had died at day 21 in medium, as evidenced by the scatter profile, and cell cycle analysis showing predominantly dying hypodiploid cells (Fig 1b). IL24 also slightly enhanced thymidine incorporation and was equally effective in cells cultured with either autologous serum or with FCS. In one sample, cells survived for up to 45 days in IL24 vs 21 days in medium.

In brief, the effect of rIL24 on cell survival was similar to that of endogenous Mda7/IL24 protein. It was next investigated if IL24 synergized with signals known to induce CLL cell proliferation.

### 1.2 rIL24 inhibits thymidine incorporation of activated CLL B-lymphocytes.

Peripheral blood samples from 33 patients with CLL were cultured for 3 days in medium or medium+IL2, with or without IL24 before 3H-thymidine incorporation. As expected, most cell samples (22/33) proliferated in response to 50 ng/ml of IL2 alone (Lantz et al. J. Exp. Med. 1985. 161: 1225-1230; Touw and Lowenberg Blood. 1985. 66:237-40), albeit with variable intensities, IL2 thymidine incorporation index ranging from 3 to >200. Table 2 shows the results from 17 patients with 8.9 to 205 fold proliferation index in comparison to cells cultured in medium. In contrast to IL2, r-IL24 alone had no detectable effect on cell proliferation at doses as high as 300 ng/ml. However, 100 ng/ml of IL24 were able to reverse partially the IL2-induced proliferation in 14/17 samples, with inhibition ranging from 11.7% (patient 11) to 77.3% (patient 29). In 3 cases (patients 4,19 and 33e), IL24 was ineffective.

**Table 2: rIL24 inhibits IL2-induced thymidine incorporation in CLL B-cells.**

| **Patient no.** | **Medium** | **IL2^{a}** | **IL2 index^{b}** | **IL2+IL24^{c}** | **%Inhibition^{d}** |
|---|---|---|---|---|---|
| 1 | 98±12.2 | 1403±20 | 17.3 | 859±40 | 38.7 |
| 2 | 67±14.8 | 1288±14.8 | 19.2 | 1088±87 | 15.5 |
| 3 | 38±22 | 591±93.6 | 15.5 | 456±196.8 | 22.8 |
| 4 | 79±22.6 | 1042±55.6 | 13.1 | 1112±28.5 | 0 |
| 5 | 91±35 | 812±12 | 8.9 | 553±137 | 31.8 |
| 11 | 357±58 | 11050±207 | 30.9 | 9755±415 | 11.7 |
| 12 | 98±31 | 2917±274 | 30.3 | 1915±232 | 34.3 |
| 13 | 111±18 | 4160±405 | 37.4 | 2130±112 | 48.8 |
| 14 | 113±30 | 3298±102 | 29.2 | 2165±210 | 34.2 |
| 16 | 91±11 | 17154±313 | 151 | 13916±2474 | 18.8 |
| 17 | 209±82 | 5588±709 | 26.7 | 4246±402 | 24.0 |
| 19 | 140±12.6 | 2225±154 | 15.9 | 2239±136 | 0 |
| 20 | 282±57 | 27020±3179 | 95.8 | 15051±4169 | 44.3 |
| 21 | 959±220 | 78283±1617 | 81.6 | 60650±143 | 22.5 |
| 29 | 73±1.6 | 3506±1124 | 48.0 | 794±187 | 77.3 |
| 30 | 172±32 | 35308±1596 | 205 | 27788±2994 | 21.3 |
| 33^{e} | 153±31 | 1639±266 | 10.7 | 1576±111 | 0 |
| 33^{f} | 876±265 | 32487±1297 | 34.0 | 13338±996 | 59.0 |

| | | | | | |
|---|---|---|---|---|---|
| a) cpm thymidine incorporation / 10⁵ CLL cells after 3 d culture with IL2. b) IL2 index reflects the ratios of IL2/medium counts. c) Cells were stimulated with IL2 overnight then with IL24 for 23 more days before thymidine incorporation. d) % inhibition was calculated from IL2+IL24/IL2 cpm ratios. e-f) The effect of IL2 and IL24 was analyzed in the same patient at CLL stage (e) and Large cell lymphoma stage (f). Cells were thawed and cultured in parallel. | | | | | |

Interestingly patient 33 underwent a Richter transformation, evolving from a CLL (33e) towards a large cell lymphoma (32f) that retained high CD5 expression and displayed an enhanced phosphorylation profile on tyrosine. Cells frozen at both CLL and lymphoma stages were thawed and studied together in the same experiment. Lymphoma cells proliferated much better spontaneously in medium alone and under IL2 than CLL cells from the same patient. IL24 inhibited both the spontaneous and IL2-induced cell growth of lymphoma cells by 59% whereas it was ineffective at the CLL stage (Table 2).

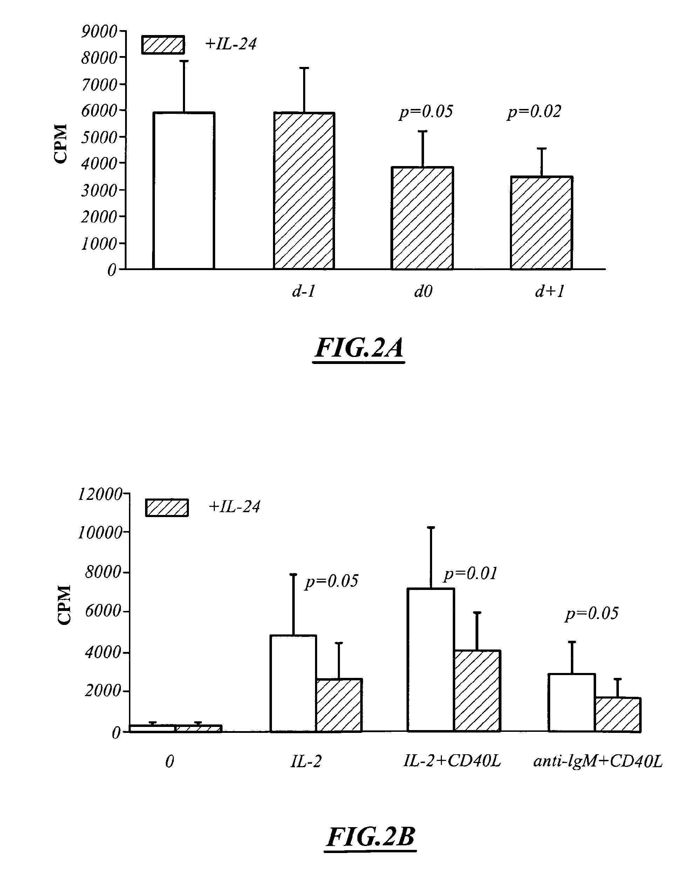

In other experiments IL24 was added to the cultures at various times before or after IL2 (**Fig 2a**). IL24 inhibitory effect was optimal if IL24 was added one day after IL2 (p=0.025) rather than at the same time (p=0.05). In contrast IL24 was ineffective if added to the culture one day before IL2.

IL24-inhibitory effect was also observed in cells stimulated with IL2+CD40-ligand or anti-IgM+CD40-ligand (**Fig 2b**) and comparable to cells stimulated with IL2 alone. Altogether, these data suggested that IL24-mediated activity predominated on stimulated/proliferating cells but not on resting cells.

### 2.3 IL24 induces apoptosis of B-cells engaged into the cell cycle.

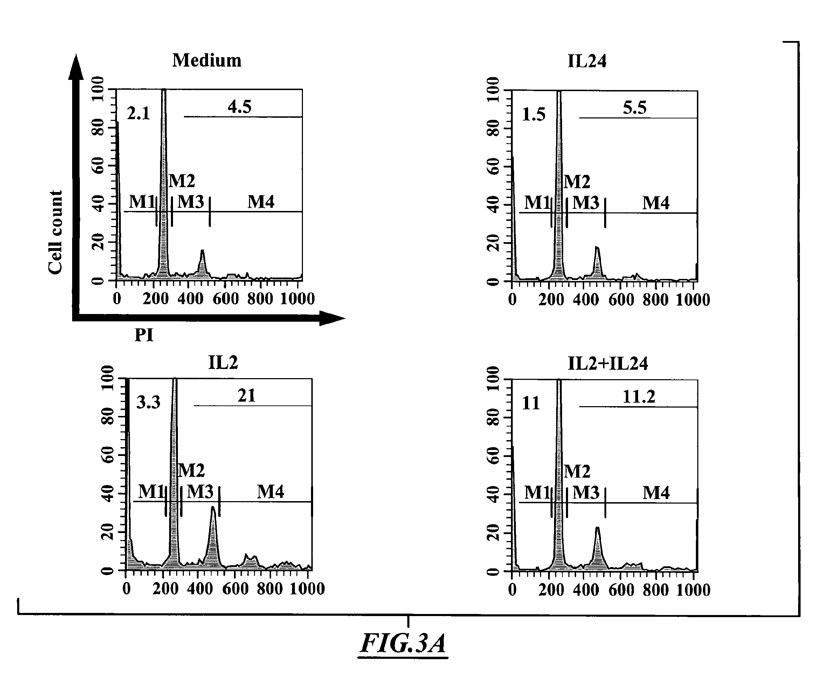

It was next determined whether the inhibition of thymidine incorporation by B-cells reflected cell cycle block and/or apoptosis. For cell cycle assays, CLL B-cells were synchronized with cold thymidine for 24 h and cultured with or without IL2, then washed and stimulated or not with IL24 for an additional 24h, fixed and stained thereafter with Propidium Iodide (PI) for FACS analysis. The respective percentages of hypodiploid (apoptotic) cells, and of cells in G0-G1, S and G2-M cell cycle phase, and finally of hyperdiploid cells were evaluated (M1 to M4 populations respectively in figure 3a). The number of cycling (S+G2/M+hyperdiploid) cells augmented fourfold under IL2 whereas IL24 alone had no significant effect as shown from one representative sample (**Fig 3a**). By contrast, the number of (S+G2/M+hyperdiploid) cells was reduced by half under IL2 + IL24. A parallel increase in hypodiploid (apoptotic) cells was observed under IL2 + IL24 (**Fig 3a**).

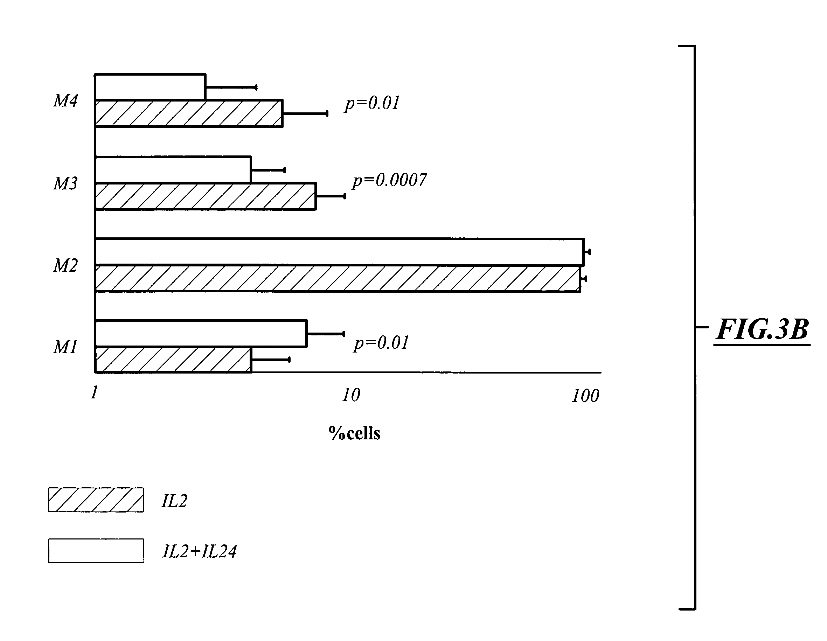

Results are summarized in figure 2b showing mean ± SD % of cells into each cycle phase. Analysis of 24 samples shows that the most significant inhibitory effect of IL24 is on the G2/M population (7.34±2.30 vs 4.01±1.4, p=0.0007) as compared to hyperdiloid cells (5.42±2.85 vs 2.71±1.58, p=0.01). In contrast, IL24 significantly augmented hypodiploid cells (4.0±1.7 vs 6.79±2.8, p=0.01) whereas the population in G0/G1 was marginally augmented (83.4±5.3 vs 87.2±5.3, p=0.1) (**Fig 3b**).

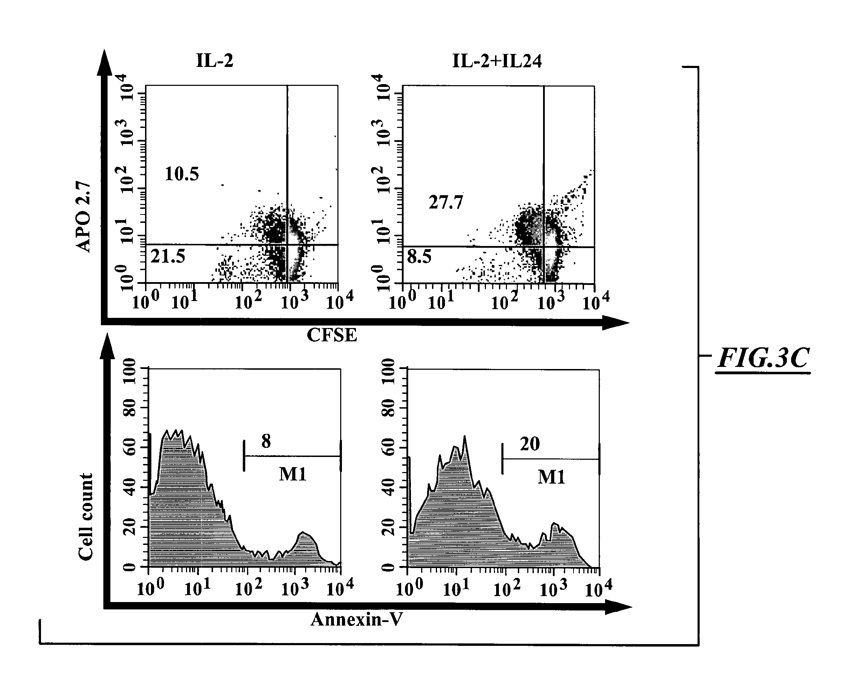

These results suggested that apoptosis occurred at the expense of proliferating cells. To verify this, cells were incubated with CFSE and cultured with IL2 with or without IL24 and stained with Apo 2.7 MAb after 2 days. Apoptosis (27.7 %) was evidenced in cells that had been stimulated with IL2 + IL24 and underwent one division (CFSE low cells) but not in cells that did not divide (CFSE high cells) (**Fig 3c**). In contrast, the number of apoptotic cells was much lower (10.5 %) in cells that had divided and had been cultured with IL2 alone (**Fig 3c**). The percentage of annexin-V-positive apoptotic cells also augmented under IL2+IL24 (20%) vs IL2 (8%) as shown from on representative sample (**Fig 3c**).

### 2.4 IL24 induces apoptosis through enhancement of p53 expression.

It was investigated whether IL24-mediated apoptosis was caspase and/or p53 dependent. The p53 protein is a transcription factor potently inhibiting cell growth, arresting cell cycle progression at G1-S and G2-M checkpoints (Levine. Cell. 1997. 323-331; Vousden. Biochim Biophys Acta. 2002. 1602-47), the expression of which is reduced in many tumors relative to normal tissues (Raman et al. Nature. 2000. 405: 974-78) and the loss associated with malignant progression (Honda et al. Blood. 2000. 95:1144-1150).

CLL cells were incubated with various combinations of IL2, IL24, ZVAD (caspase inhibitor), and Pifithrin (pft-a), an inhibitor of p53 transactivation (Komarov et al. Science. 1999. 85:1733-37), and cell cycle analysis was performed on cells stained with PI. It was observed that the number of cells in G2/M cell cycle phase was reduced by half under IL2 + IL24 as compared to IL2 (3.36 ± 1.3% vs 7.1± 1.5%, p=0.03). Pft-a almost totally reversed the diminution of cells in S/G2/M induced by IL24 (6.44 ± 2.2% vs 3.36 ± 1.3%, p=0.03) (**Fig 4****, a and b**). The effect of IL24 on S/G2/M cells was also neutralized to a lesser extent by zvad (IL2+IL24+zvad: 4.8 ± 1.77%, p=0.16) (**Fig 4****, a and b**).

These results suggested that cells driven to proliferation by IL2 underwent a p53-mediated cell cycle block followed by -at least partly- caspase-dependent apoptosis. This was further confirmed by the observation that IL24 augmented p53 expression in CLL cells. As shown by western blots and quantitative (q) PCR (**Fig 4c, 4d**), p53 protein and transcripts augmented slightly after 2 days of stimulation with IL24 alone and significantly after stimulation with IL2+IL24 as compared to non stimulated and IL2-stimulated cells respectively. Contrary to p53, we failed to observe any change in bcl2 and p38MAPK protein expression (**Fig 4c**). The Phosphorylation on serines in the N terminal transactivation domain of p53 were shown to be crucial for p53 expression and stabilization (Toledo et al. Nat Rev Cancer. 2006. 6:909-23). Indeed an increase in P-ser15-p53 was observed in IL2+IL24-stimulated cells (**Fig 4c**).

Thus IL24 promoted the expression and possibly the stabilization of p53 protein, in turn acting as a cell cycle regulator. In further support of this, downstream targets of p53, such as p19, p21 (el-Deiry et al. Cell. 1993. 19:817-25), and GADD-45 (Zhan et al. Cancer Res. 1994. 54:2755-60) gene products, were enhanced by IL24 after 72h, whereas, by contrast, MDM2, a molecule that helps degrade p53 (26,39) was inhibited as shown by qPCR (**Fig 4d**).

### 2.5 IL24 suppresses IL2-induced stat3 phosphorylation through a phosphatase-dependant mechanism.

It has been demonstrated that oncogenic pathways that signal through Stat3 inhibit p53 expression (Niu et al. Mol Cell Bio. 2005. 25: 7432-40). Stat3 is a latent transcription factor activated on tyrosine following interaction with several growth factors and cytokines (Zhong et al. Science. 1994. 264:95-8) including cytokines of the gamma-c family such as IL2, and translocating thereafter into the nucleus (Niu et al. Mol Cell Bio. 2005. 25: 7432-40). Later on, it was shown that all members of the class-II family of cytokines which includes IL24 (Renauld JC. Nat Rev Immunol. 2003. 3:667-76; Zhong et al. Science. 1994. 264:95-8; Dumoutier et al. J Immunol. 2001. 167:3545-49; Wang et al. Immunology. 2005. 114:166-170) activated stat3 although studies were performed on non-lymphoid cells.

Thus, the finding that IL24 activated stat3 would have been in contradiction with our observation that IL24 enhanced p53 expression in CLL. Finally, IL24-mediated apoptosis was reported to be independent on stats in several non-lymphoid cell lines (Sauane et al. J Cell Physiol. 2003. 196:334-345). Yet, our results suggested that IL24 interfered with the IL2 signaling pathway.

IL2 signaling in CLL was therefore analyzed. Protein extracts from cells incubated with IL2 were immunoprecipitated with anti-phosphotyrosine 4G10 MAb and western blots performed with MAbs specific for proteins of the Jak/stat pathway. Following interaction with IL2, IL2-R is phosphorylated and triggers the phosphorylation of Jak1 which associates with IL2-Rb chain which associates with gamma-c chain (Friedmann et al. Proc Natl Acad Sci USA. 1996. 93:2077-82), and finally activate STAT5 and STAT3 (Friedmann et al. Proc Natl Acad Sci USA. 1996. 93:2077-82, Frank et al. Proc Natl Acad Sci USA. 1995. 92:7779-83).

IL2 induced a rapid phosphorylation (within 2 min), that peaked at 10 min, of Jak1, Tyk2, stat3 and stat5 proteins on tyrosine residues, in CLL cells. Thus, IL2 signaling in CLL cells involves the phosphorylation of jak/stat proteins similar in this respect to that reported in lymphocytes and lymphoid cell lines.

Next, we analyzed cells cultured with IL2 overnight and stimulated or not with IL24, the latter condition which was shown to induce apoptosis. Stat3 was strongly and stably phosphorylated in cells incubated overnight with IL2 as evidenced by hybridization with an anti Phospho-Y705 stat3 MAb. In contrast, phospho(P)-stat5 disappeared and total stat5 diminished in the cytosol of cells incubated overnight with IL2, presumably due to dimerization and translocation to the nucleus (Paukku et al. Cytokine Growth Factor Rev. 2004. 6:435-55).

The addition of 100 ng/ml of rIL24 to IL2-stimulated cells almost totally inhibited P-stat3 within 2 min, and P-stat3 remained undetectable for the 30 min duration of the experiment, whereas the amount of total stat3 remained unchanged in the cytosol. This suggested an active, phosphatase-mediated mechanism. Cells were therefore incubated or not for 5 min with 25 mM pervanadate (PV), an inhibitor of protein tyrosine phosphatase activity (Heffetz and Bushkin H. J Biol Chem. 1990. 265:2896-902) and stimulated 10 min with IL24 on cells preincubated or not with IL2. Cytospin and western blot experiments were performed. While P-Stat3 was induced by IL2 and rapidly inhibited by IL24, PV antagonized IL24-mediated inhibition of P-Stat3. The same conclusions could be drawn from immunostaining experiments showing that P-stat3 was extinguished in IL2-cultured cells, by IL24 and restored by PV, but did not relocate in the nucleus.

### 2.6) Troglitazone induces p53 expression, stat3-dephosphorylation and apoptosis of CLL cells.

Our results demonstrated that IL24-induced apoptosis in CLL is dependant on p53 expression, and thus suggested that the latter depended on P-stat3 dephosphorylation, since P-stat3 is a repressor of p53 transcription. To further strengthen this hypothesis, pharmacological activators or inhibitors of phosphatases were used in the experiments. Indeed, PV is too toxic to be used in culture for > 30 min.

Troglitazone (TG) is an activator of protein-tyrosine phosphatase (PTP)-1B that reduced P-Y705-stat3 and promoted apoptosis in human primary gliomas (Akasaki et al. J Biol Chem. 2005. 281:6165-74). TG was therefore used to determine if it is able to stimulate p53 expression and apoptosis in CLL. It was found that TG induced a dose response dephosphorylation of P-stat3 in IL2-cultured CLL cells and in parallel an increase in p53 protein, but had no effect on total Stat3 expression (**Fig 5a**). TG also induced the death of IL2-stimulated cells as shown by cell cycle analysis (**Fig 5b**) and annexin staining and cell count. Of note, whether IL24 killed cells in S/G2M and spared cells in G0/G1, the latter population was predominantly affected by TG. Thus this molecule had similar, albeit distinct, cellular and molecular effects as IL24. However it supported the observation that the inhibition of P-stat3 is a general mechanism leading to enhanced p53 expression and cell death in proliferating cells.

CLL cells were next incubated with two phosphatase inhibitors in order to antagonize the effects of IL24. Cells stimulated with IL2 overnight were treated respectively with two inhibitors of SHP-1 and PTP1B (PTPI1) or SHP-1 only (PTPI3), 60 min before stimulation with IL24. This supported the notion that SHP-1 is a target for IL24.

### 2. 7 SHP-1 associates to the IL24 receptor in CLL cells and is recruited to the Receptor following interaction with IL24.

Two src homology-2 (SH2) containing tyrosine phosphatases 1 (SHP-1) and 2 (SHP-2) are recruited by several haematopoietic surface receptors including IL2 Receptor beta chain (Adachi et al. Oncogene. 1997. 14:1629-33, Migone et al. Proc Natl Acad Sci USA. 1998. 95:384-50). Consequently, phosphatases play a role in terminating Interleukin-mediated signals by dephosphorylating Tyk and Jak proteins upstream stat and even by directly dephosphorylating stat proteins (Haspel et al. EMBO J. 1996. 15:6262-70).

It was observed that CLL cells express high amounts of SHP-1 protein. Cells, stimulated or not with IL24 and IL24 receptor chains, were immunoprecipitated in protein extracts, using a mixture of anti-IL20 R-a and anti IL20 R-b Abs or of isotype-matched controls, followed by SDS PAGE and Western blot with anti SHP-1 Ab. SHP-1 was found to associate naturally to the IL24 receptor. Moreover, this association is augmented in cellular extracts from cells incubated with rIL24 for 10 minutes, but not if cells were incubated with TG, Therefore, engagement of the IL24 R by its ligand results in the recruitment of SHP-1 to IL24 Receptor chains.

### Example 3: IL24 induces death of SP2/0 myeloma cells stimulated by IL-6

SP2/0 cell line is a fusion partner commonly used to generate hybridomas in order to make monoclonal antibodies. Although this cell line is factor independent, it was observed that it is still able to respond to IL-6 as it incorporates more thymidine following IL-6 exposure. This was somewhat expected since IL-6 is a myeloma growth factor.

In this experiment, cells were cultures with medium (RPMI-10%FCS), optionally supplemented with 20 ng IL-6 and/or 100 ng IL24.

Cell cycle analyses were performed as described in Example 1, by labelling with Propidium Iodide and FSC, and fluorescence measurement using a FACScan flow cytometer (BD Biosciences). Data analysis was performed using CellQuest software (BD Biosciences). The results are shown in Table 3, below.

**Table 3: IL24 induces death of SP2/0 myeloma cells stimulated by IL-6**

| Cell cycle phase | Medium | Medium + IL-6 | Medium + IL24 | Medium + IL-6, then Medium + IL24 |
|---|---|---|---|---|
| G0/G1 | 41.7% | 34.1% | 36.4 % | 24.8% |
| S-G2/M | 32.1% | 37.5% | 39.9% | 31.5% |
| hypodiploid | 26.2% | 28.4% | 23.7% | 43.7% |

It was found that 32.1 % of the SP2/0 cells cultured with medium enter the cell cycle (S-G2/M) spontaneously, whereas 41.7% are into G0/G1.

Cells into S-G2/M cell cycle phase increased to 37.5% and 39.9%, respectively, when cultured with medium containing IL-6 and with medium containing IL24. A parallel decrease of the population in G0/G1 was observed: 34.1 % (medium containing IL-6) and 36.4 % (medium containing IL24).

Thus, IL24 and IL-6 enhance proliferation and/or survival.

When cells were cultured overnight with IL-6, then with IL24 for 2 more days, both populations in G0/G1 and S/G2/M phases are inhibited (24.8 and 31.5% respectively) as compared with IL24 or IL-6 alone, and even as compared with medium. Hypodiploid (apoptotic) cells represent 43.7% of total cells indicating that sequential addition of IL6 and IL24 is detrimental to cell survival.

This work enabled to describe for the first time the conditions under which IL24 exerts a pro-apoptotic effect on CLL and to dissect molecular events associated with this function. Until now, the effect of IL24 on cell death was observed following adenoviral delivery of mda7 into cell lines and said to be independent on IL24-R (Sauane et al. J Cell Physiol. 2003. 196:334-345).

Since it was observed that intracellular Mda7/IL24 is highly expressed in CLL cells at the mRNA and protein levels, it was tempting to study the effect of the exogenous cytokine, inasmuch as CLL cells express IL24-receptor chains (Sainz-Perez et al. Leukemia. 2006. 20:498-504). rIL24 was found to enhance the survival of CLL cells in culture thus mimicking the effect of endogenous Mda7/IL24. This supported our previous data in which silencing of Mda7/IL24 RNA induced apoptosis and inhibited natural p38-MAPK phosphorylation in CLL cells whereas rIL24 induced p38-MAPK phosphorylation in these cells (Sainz-Perez et al. Leukemia. 2006. 20:498-504).

These results seemed at odds with the published apoptotic effect of adeno-mda7 (Ekmekcioglu et al. Int J Cancer. 2001 Oct 1;94(1):54-9 ; Lebedeva et al. Oncogene. 2002. 21:708-18 ; Su et al. Proc Natl Acad Sci U S A. 1998. 95:14400-5). The finding that rIL24 is proapoptotic provided CLL cells are stimulated in order to enter the cell cycle, reconcile with these results.

Mda7 has been cloned originally in differentiated cells and its expression inversely correlated with the proliferation of malignant melanocytes (Ellerhorst et al. J Clin Oncol. 2002.20:1069-74 ; Ekmekcioglu et al. Int J Cancer. 2001 Oct 1;94(1):54-9). It is therefore comprehensible that Mda7/IL24 is protective in terminally differentiated cells, which is the case in CLL (Klein et al. J Exp Med. 2001. 194:1625-1638; Rosenwald et al. J Exp Med. 2001. 194:1639-47), or conversely, that IL24, whether delivered through adenovirus or as a cytokine, kills proliferating cell lines or IL2-stimulated cells. In CLL, the death mechanism is clearly linked to the stimulation of p53 mRNA and protein expression, as it is totally reversed by a pharmacological inhibitor of p53. Caspases are also involved to some extent in this process and need to be studied more in depth.

P53 is central in the control of tumour progression and often downregulated or rendered inactive by mutation (Toledo et al. Nat Rev Cancer. 2006. 6:909-23; Efeyan and Serrano. Cell cycle. 2007. 6:1006-1010; Sherr. Cell. 2004. 116:235-246). Recently it has been demonstrated that restoring endogenous p53 even for a brief period induced regression of murine liver carcinomas *in vivo,* not by inducing apoptosis but instead by induction of a senescence program associated with the production of inflammatory cytokines such as M-CSF, CCL2, CXCL1, IL15 (Xue et al. Nature. 2007. 445: 656-60). It was also demonstrated that restoration of p53 function *in vivo* led to tumor regression with induction of cellular senescence in sarcomas and apoptosis in lymphomas (Ventura et al. Nature. 2007. 445: 661-5).

Since ZVAD could not reverse death completely, both senescence and apoptosis mechanisms may be at work in CLL. These models are in agreement with the data shown herein and pave the way for the use of p53 activators to treat human cancers. P53 accumulates in cells following stress signals that stop its degradation and/or enhance its transcription and its activity as a transcription factor (Toledo et al. Nat Rev Cancer. 2006. 6:909-23) as shown by the regulation of several downstream genes (Figure 4). IL24 can be viewed here as a stress signal that turns off the IL2-signaling cascade and induces p53 expression, likely by inactivating the p53-transcriptional repressor P-Stat3. Yet other mechanisms may be operating as well through the stabilization of p53 and/or inhibition of its degradation. The latter involve the phosphorylation of p53 on Ser15 by at least 8 different Ser/Thr kinases (Toledo et al. Nat Rev Cancer. 2006. 6:909-23). Among them, p38 MAPK was shown to be activated by IL24 (Sainz-Perez et al. Leukemia. 2006. 20:498-504).

This role of P-Stat3 dephosphorylation on p53 derepression and downstream induction of cell death is supported by our experiments using pharmacological activators or inhibitors of phosphatases. TG, an antidiabetic drug and a PPAR-g agonist (Akasaki et al. J Biol Chem. 2005. 281:6165-74) that inhibits the tyrosine phosphatase PTP1B, dephosphorylated P-Stat3 and augmented p53 expression and apoptosis in IL2-stimulated CLL cells, thereby mimicking the effect of IL24. Conversely, Pase inhibitors reversed the effects of IL24, and augmented P-Stat3 in CLL. Thus, inhibiting P-Stat3 to enhance p53 expression, which in the context of proliferating cells is sensed as a danger signal and triggers cell death, may be a general mechanism operating in normal and malignant cells as well. This implies that p53 is not made inactive by mutation or deletion and can act as a transcription factor. This is indeed the case in CLL (Thornton et al. Hematol J. 2004. 5:47-54; El rouby et al. Blood. 1993. 82:3452-9; Kojima et al. Blood. 2006. 108:993-1000) and in most cancers since mutations generally occur late during evolution (Raman et al. Nature. 2000. 405: 974-78 ; Baker et al. Cancer Res. 1990. 50:7717-722 ; Pharoah et al. Br J Cancer. 1999.80:1968-73).

Given that IL24 was shown to induce Stat3 phosphorylation in epithelial cell lines and keratinocytes (Dumoutier et al. J Immunol. 2001. 167:3545-49, Kunz et al. Exp Dermatol. 2006. 15:991-1004), the inhibition of Sta3 phosphorylation in CLL cells by IL24 was quite unexpected, but nevertheless provided a molecular explanation of IL24-mediated cell death. Interestingly, Stat3 is naturally phosphorylated at a low level on tyrosines in CLL This phosphorylation was always slightly inhibited by IL24 alone, whereas it was activated, by IL2. The contradiction between our results and others may be due to the nature of the cells. Indeed IL24 failed to activate Stat3 in PBMC (Kunz et al. Exp Dermatol. 2006. 15:991-1004) but this was attributed to the lack of functional IL24-R as only the common IL20-R2 receptor-chain was detected by q-PCR. Instead our previous data showed that all three chains were amplified in CLL and at least one (IL24-R1) receptor dimer was evidenced by FACS (Sainz-Perez et al. Leukemia. 2006. 20:498-504). Differences in phosphatase expression and localization between cell types may therefore explain these differences.

In physiological conditions, SHP-1 is recruited to the IL2-Rb and dephosphorylate proteins of the Jak/stat family in order to terminate the IL2-induced signal. SHP-1 was indeed found to associate with IL2-Rb. We indeed failed to observe a dephosphorylation of Jak1 and Tyk2 proteins under IL24. Importantly, SHP-1 is present in huge amounts in CLL and is recruited to the IL24-R following stimulation with IL24. It is therefore possible that if IL2- and IL24-Receptors reside in the same microdomains, SHP-1 is brought close to Stat3 and terminates the IL2-mediated signal.

## Claims

1. IL24 for the treatment of a hyperproliferative or autoimmune disorder in a patient in need thereofby inducing death of cycling hyperproliferative or autoimmune cells.

2. IL24 for the treatment according to claim 1, wherein said cycling hyperproliferative or autoimmune cells have been triggered into cell cycle beforehand by administering said patient with a cytokine.

3. IL24 for the treatment according to claim 1 or 2, wherein death of cycling hyperproliferative or autoimmune cells is induced by stimulating p53 expression.

4. IL24 for the treatment according to any one of claims 1 to 3, wherein said cycling hyperproliferative or autoimmune cells are Stat3-activated cells.

5. IL24 for the treatment according to any one of claims 1 to 4, wherein death of said cycling hyperproliferative or autoimmune cells is induced by derepression of p53 expression.

6. IL24 for the treatment according to claim 5, wherein derepression of p53 expression is induced by inhibiting activated-Stat3.

7. IL24 for the treatment according to claim 5 or 6, wherein derepression of p53 expression is induced by dephosphorylating phospho-Stat3 (P-Stat3).

8. IL24 for the treatment according to any one of claims 1 to 7, wherein death of cycling hyperproliferative or autoimmune cells is achieved by apoptosis and/or senescence.

9. IL24 for the treatment according to any one of claims 1 to 8, wherein said hyperproliferative disorder is cancer.

10. IL24 for the treatment according to claim 9, wherein said cancer is selected from the group consisting of acute myelogenous leukaemia, chronic lymphocytic leukaemia, multiple myeloma, Hodgkin's disease, non-Hodkin's lymphoma, B cell, cutaneous T cell lymphoma, brain, lung, breast, ovarian, head and neck, bladder, gastric, pancreatic, renal, prostate, colorectal, oesophageal, thyroid cancer, and melanoma.

11. IL24 for the treatment according to claim 9 or 10, wherein said cancer is chronic lymphocytic leukaemia.

12. IL24 for the treatment according to claim 11, wherein said patient was administered beforehand with IL2.

13. IL24 for the treatment according to claim 9 or 10, wherein said cancer is myeloma.

14. IL24 for the treatment according to claim 13, wherein said patient was administered beforehand with IL-6.

15. IL24 for the treatment according to any one of claims 1 to 8, wherein said cycling autoimmune cells are autoimmune B cells.

16. IL24 for the treatment according to claim 15, wherein said autoimmune disorder is selected from the group consisting of systemic erythematosus Lupus, Sjogren's syndrome and rheumatoid arthritis.

17. A kit comprising IL24 and a cytokine likely to trigger a hyperproliferative or autoimmune cell type into cell cycle, as a combined preparation for sequential use for the treatment of a disorder associated with said hyperproliferative or autoimmune cell.

18. The kit according to claim 17, wherein said cytokine induces Stat3 phosphorylation.

19. The kit according to claim 17 or 18, wherein said cytokine is IL2 and wherein the kit is intended the treatment of chronic lymphocytic leukaemia.

20. The kit according to claim 17 or 18, wherein said cytokine is IL-6 and wherein the kit is intended for the treatment of myeloma.

21. An inhibitor of activated-Stat3 for treating chronic lymphocytic leukaemia (CLL).

22. An inhibitor of activated-Stat3 according to claim 21, wherein CLL cells were beforehand contacted with IL2.

23. An inhibitor of activated-Stat3 according to claim 21 or 22, wherein said inhibitor of activated-Stat3 is selected from the group consisting of IL24, troglitazone, PIAS3, 6-nitrobenzo[b]thiophene-1,1-dioxide, cucurbitacin I, cucurbitacin Q, Withacnistin, curcumin, and AG490.
